# EUROPÄISCHE PATENTSCHRIFT

(11) **EP 1 483 438 B1**
(45) Veröffentlichungstag und Bekanntmachung des Hinweises auf die Patenterteilung: **14.02.2007**
(21) Anmeldenummer: 03742947.9
(22) Anmeldetag: 20.02.2003
(51) Int. Cl.: D06L 3/12, C07D 231/06, C08K 5/3445

(54) **WÄSSRIGE FLÜSSIGFORMULIERUNGEN VON PYRAZOLIN-AUFHELLERN**
AQUEOUS LIQUID FORMULATIONS OF PYRAZOLINE BRIGHTENERS
FORMULATIONS LIQUIDES AQUEUSES D'AGENTS DE BLANCHIMENT OPTIQUE A BASE DE PYRAZOLINE

(30) Priorität: 28.02.2002 DE 10208773
(43) Veröffentlichungstag der Anmeldung: 08.12.2004
(73) Patentinhaber: Clariant Produkte (Deutschland) GmbH, 65929 Frankfurt am Main (DE)
(72) Erfinder: ZIRKENBACH, Gerhard, 65385 Rüdesheim (DE); LERCH, Alexander, 63571 Gelnhausen (DE)
(86) Internationale Anmeldenummer: PCT/EP2003/001730
(87) Internationale Veröffentlichungsnummer: WO 2003/072870

(56) Entgegenhaltungen:
- EP-A- 0 073 996
- US-A- 3 925 367

## Beschreibung

Es ist bekannt, bestimmte Pyrazolin-Aufheller, die basische Gruppen tragen und in Form ihrer Salze mit Milchsäure vorliegen, zum Aufhellen von Acrylfasern zu verwenden (DE 3 134 942). Kommerziell erhältlich ist eine wasserhaltige Flüssigformulierung eines derartigen Aufhellers, die neben dem Aufheller noch Milchsäure, Ameisensäure und Methoxypropanol enthält. Dabei handelt es sich um das Produkt BLANKOPHOR^{®} DRS von Bayer. Diese Formulierung ist jedoch in Bezug auf ihre Lagerbeständigkeit noch verbesserungsfähig. Die Aufgabe der Erfindung besteht somit darin, wässrige Flüssigformulierungen von Pyrazolin-Aufhellern zur Verfügung zu stellen, die eine verbesserte Lagerstabilität aufweisen. Es wurde nun gefunden, dass man dieses Ziel erreichen kann, indem man der Formulierung dieses Aufhellertyps einen verzweigten mono- und di-Alkohol zusetzt.

Gegenstand der Erfindung sind
wässrige Flüssigformulierungen von Pyrazolin-Aufhellern der Formel 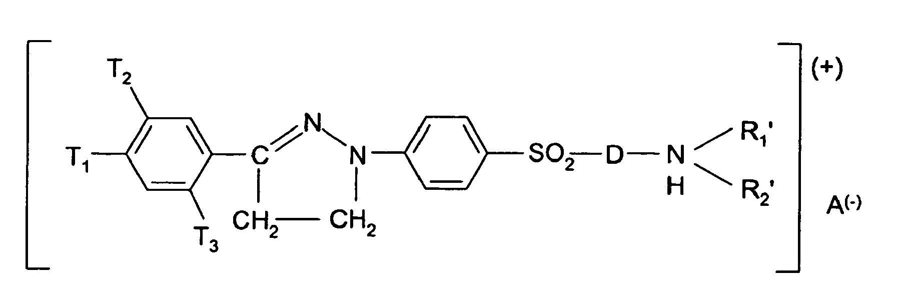 worin
- D: -CH₂CH₂-, -CH₂CH₂CH₂-, -CH₂CH₂OCH₂CH₂, 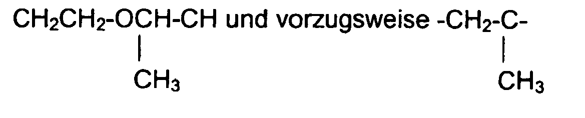
- R₁' und R₂': C₁-C₂-Alkyl,
- T₁, T₂, T₃: H, CH₃ oder Cl und
- A: ein Anion einer organischen Säure bedeuten,
wobei diese Flüssigformulierungen neben einem Pyrazolin-Aufheller der obigen Formel eine organische Säure und Neopentylglykol enthalten.

Als Anion A⁽⁻⁾ kommen in Frage die Anionen niedermolekularer organischer Säuren, beispielsweise Formiat und Lactat.

Vorzugsweise stehen T₁-T₃ nicht gleichzeitig für Cl oder CH₃.

Die Menge dieser Pyrazolin-Aufheller in der fertigen Flüssigformulierung kann 1 bis 60, vorzugsweise 5 bis 30 Gew.-% betragen.

Die Pyrazolinbasen, die den oben definierten Aufheller-Salzen zugrunde liegen, sind bekannt und beispielsweise in folgender Patentliteratur direkt oder indirekt (als Quartärsalze) beschrieben:

DE-A-1 155 418, 1 237 124, 1 469 222, 1 904 424, 2 011 552, 2 050 725, 2 248 772, 2 534 180 und 2 700 996 sowie US-A-3 131 079 und 3 135 742.

Neben dem Aufheller-Salz enthalten die erfindungsgemäßen Flüssigformulierungen noch organische Säuren und Neopentylglykol. Als organische Säuren kommen in Frage niedermolekulare organische Säuren, wie sie zur Salzbildung der Pyrazolinbasen üblich sind, beispielsweise Ameisensäure, Essigsäure und Milchsäure oder Gemische solcher Säuren. Die Menge dieser Säuren, bezogen auf die fertige Flüssigformulierung, beträgt im allgemeinen 20 bis 60, vorzugsweise 30 bis 50 Gew.-%.

Der Anteil des Neopentylglykols in der fertigen Flüssigformulierung kann 15 bis 40, vorzugsweise 20 bis 30 Gew.-% betragen.

Darüber hinaus können die erfindungsgemäßen Flüssigformulierungen noch die bei optischen Aufhellern üblichen Hilfsstoffe enthalten, wie beispielsweise hydrotope Mittel (z. B. Harnstoff), lösungsstabilisierende Mittel (z. B. Ethylenglykoldiacetat), Konservierungsmittel, wasserlösliche kationische Nuancierungsfarbstoffe, jeweils in Mengen bis zu 10 Gew.-%.

Die Herstellung der erfindungsgemäßen Flüssigformulierungen erfolgt vorzugsweise in der Weise, dass man die einzelnen Komponenten der Formulierung in folgender Reihenfolge zugibt und in geeigneter Weise vermischt: organische Säure, Neopentylglykol, Aufhellersalz, Wasser. Anstelle des Aufhellersalzes kann man auch von der zugrundeliegenden Base ausgehen und erhöht die Menge an organischer Säure um die zur Salzbildung benötigte Menge.

Die erfindungsgemäßen Flüssigformulierungen zeichnen sich gegenüber der bisher üblichen Handelsform durch eine verbesserte Lagerstabilität aus. Diese verbesserte Lagerstabilität kommt dadurch zum Ausdruck, dass die Farbwerte dieser Formulierungen insbesondere auch nach längerer Lagerung deutlich geringer ansteigen.

In an sich bekannter Weise werden die erfindungsgemäßen Flüssigformulierungen zur Einarbeitung in Spinnmassen für die Herstellung von Polyacrylnitritfasern im sogenannten Nassspinnverfahren eingesetzt oder zum Aufhellen von fertigen Polyacrylfasern in einem Ausziehverfahren.

### Beispiele

Es wurde eine Flüssigformulierung hergestellt, indem bei Raumtemperatur folgende Komponenten in dieser Reihenfolge intensiv gemischt wurden:
40 g Milchsäure; 6 g Ameisensäure; 23,3 g Neopentylglykol (90 %ig); 19,2 g Aufheller (ca. 79 %ig); 11,5 g Wasser. Der benutzte Aufheller hat die Formel

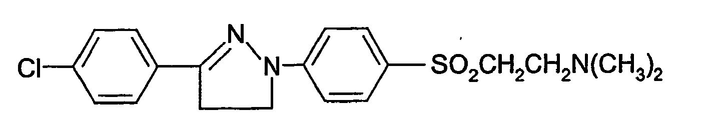

Diese Flüssigformulierung wurde gemeinsam mit einer handelsüblichen Flüssigformulierung des gleichen Aufhellers auf Lagerstabilität (Messung der Farbwerte x und y) untersucht. Die handelsübliche Flüssigformulierung enthält 21 g Methoxypropanol und 13,8 g Wasser anstelle von 23,3 g Neopentylglykol und 11,5 g Wasser bei ansonsten gleicher Zusammensetzung wie die erfindungsgemäße Formulierung.

Bei beiden Formulierungen wurden folgende x- und y-Werte als Maß für die Stabilität gemessen:

| | Erfindungsgemäße Formulierung | | Handelsware | |
|---|---|---|---|---|
| | x-Wert | y-Wert | x-Wert | y-Wert |
| Zu Beginn | 0,353 | 0,390 | 0,354 | 0,392 |
| nach 4 Wochen Lagerung RT/dunkel | 0,362 | 0,404 | 0,368 | 0,401 |
| nach 4 Wochen Lagerung 50°C/dunkel | 0,363 | 0,402 | 0,402 | 0,425 |
| nach 10 Wochen Lagerung RT/dunkel | 0,383 | 0,433 | 0,390 | 0,433 |
| nach 10 Wochen Lagerung 50°C/dunkel | 0,386 | 0,428 | 0,449 | 0,466 |

Diese Werte zeigen, dass die Farbwerte bei der erfindungsgemäßen Formulierung deutlich geringer ansteigen als bei der Handelsware. Dies bedeutet eine bessere Stabilität der erfindungsgemäßen Formulierung mit einem Gehalt an Neopentylglykol.

## Patentansprüche

1. Wässrige Flüssigformulierungen von Pyrazolin-Aufhellern der Formel 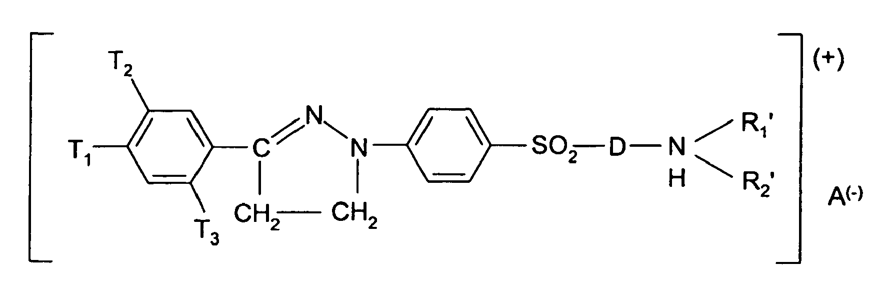 worin
D -CH₂CH₂-, -CH₂CH₂CH₂-, -CH₂CH₂OCH₂CH₂, 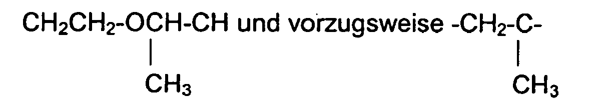
R₁' und R₂' C₁-C₂-Alkyl,
T₁, T₂, T₃ H, CH₃ oder Cl und
A ein Anion einer organischen Säure bedeuten,
**dadurch gekennzeichnet, dass** diese Flüssigformulierungen neben einem Pyrazolin-Aufheller der obigen Formel eine organische Säure und Neopentylglykol enthalten.

2. Wässrige Flüssigformulierungen nach Anspruch 1, **dadurch gekennzeichnet, dass** sie als organische Säure Milchsäure oder Ameisensäure oder Essigsäure oder deren Mischungen enthalten.

3. Wässrige Flüssigformulierungen nach Anspruch 1, **dadurch gekennzeichnet, dass** sie 20 bis 60 Gew.-% organische Säure enthalten.

4. Wässrige Flüssigformulierungen nach Anspruch 1, **dadurch gekennzeichnet, dass** sie 15 bis 40 Gew.-% Neopentylglykol enthalten.

5. Wässrige Flüssigformulierungen nach Anspruch 1, **dadurch gekennzeichnet, dass** sie 1 bis 60 Gew.-% des Pyrazolin-Aufhellers enthalten.

6. Wässrige Flüssigformulierungen nach Anspruch 1, **dadurch gekennzeichnet, dass** sie die bei Flüssigformulierungen von optischen Aufhellern üblichen Hilfsstoffe enthalten.

7. Verwendung der wässrigen Flüssigformulierungen nach Anspruch 1 zum Aufhellen von Polyacrylfasern.

## Claims

1. Aqueous liquid formulations of pyrazoline brighteners of the formula 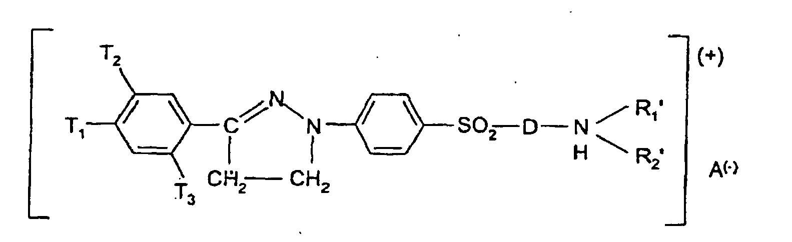 where
D denotes -CH₂CH₂-, -CH₂CH₂CH₂-, -CH₂CH₂-OCH₂CH₂, 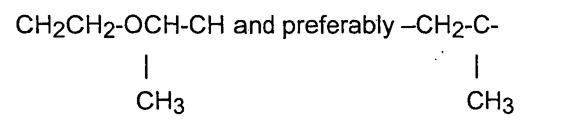
R₁' and R₂' denote C₁-C₂-alkyl, and
T₁, T₂, T₃ denote H, CH₃ or Cl and
A is an anion of an organic acid,
**characterized in that** these liquid formulations contain an organic acid and neopentylglycol as well as a pyrazoline brightener of the above formula.

2. Aqueous liquid formulations according to claim 1, **characterized in that** the organic acid they contain is lactic acid or formic acid or acetic acid or mixtures thereof.

3. Aqueous liquid formulations according to claim 1, **characterized in that** they contain 20% to 60% by weight of organic acid.

4. Aqueous liquid formulations according to claim 1, **characterized in that** they contain 15% to 40% by weight of neopentylglycol.

5. Aqueous liquid formulations according to claim 1, **characterized in that** they contain 1% to 60% by weight of the pyrazoline brightener.

6. Aqueous liquid formulations according to claim 1, **characterized in that** they contain the customary auxiliaries for liquid formulations of optical brighteners.

7. The use of the aqueous liquid formulations according to claim 1 for brightening acrylic fibers.

## Revendications

1. Formulations liquides aqueuses d'agents de blanchiment à la pyrazoline répondant à la formule 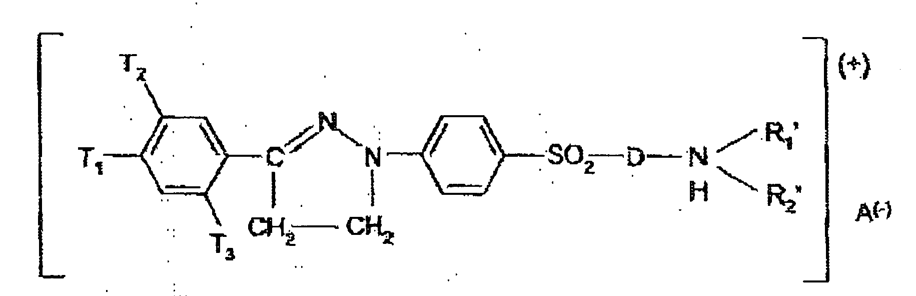 dans laquelle
D -CH₂CH₂-, -CH₂CH₂CH₂-, -CH₂CH₂OCH₂CH₂, 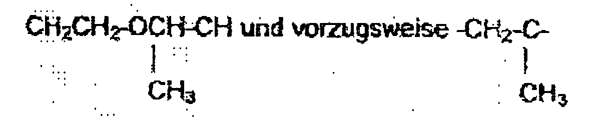
R₁' et R₂' signifient alkyle en C₁-C₂,
T₁, T₂, T₃ H, CH₃ ou Cl et
A un anion d'un acide organique,
**caractérisées en ce que** ces formulations liquides contiennent, en plus d'un agent de blanchiment à la pyrazoline répondant à la formule ci-dessus, un acide organique et du néopentylglycol.

2. Formulations liquides aqueuses selon la revendication 1, **caractérisées en ce qu'**elles contiennent comme acide organique de l'acide lactique ou de l'acide formique ou de l'acide acétique ou leurs mélanges.

3. Formulations liquides aqueuses selon la revendication 1, **caractérisées en ce qu'**elles contiennent de 20 à 60 % en poids d'acide organique.

4. Formulations liquides aqueuses selon la revendication 1, **caractérisées en ce qu'**elles contiennent de 15 à 40 % en poids de néopentylglycol.

5. Formulations liquides aqueuses selon la revendication 1, **caractérisées en ce qu'**elles contiennent de 1 à 60 % en poids de l'agent de blanchiment à la pyrazoline.

6. Formulations liquides aqueuses selon la revendication 1, **caractérisées en ce qu'**elles contiennent des adjuvants usuels dans les formulations liquides d'agents de blanchiment optiques.

7. Utilisation des formulations liquides aqueuses selon la revendication 1 pour le blanchiment de fibres de polyacryle.
